# EUROPEAN PATENT APPLICATION

(11) **EP 2 886 657 A1**
(43) Date of publication of application: **24.06.2015**
(21) Application number: 13005917.3
(22) Date of filing: 19.12.2013
(51) Int. Cl.: C12P 7/62, C12P 33/00, C12P 5/00, C12N 9/20

(54) **Enzymatic synthesis of phenolic acid esters and steryl phenolates**

(71) Applicant: ETH Zurich, 8092 Zurich (CH)
(72) Inventor: Nyström, Laura, CH - 8006 Zürich (CH); Schär, Aline, CH - 4600 Olten (CH)

(57) **Abstract**

The present invention relates to a method for obtaining at least one steryl phenolate from at least one phenolic acid in an enzymatic process using a lipase, wherein in a first step a) the at least one phenolic acid and at least one alcohol R¹-OH undergo an enzymatic esterification to at least one phenolic acid ester according to reaction scheme (III) 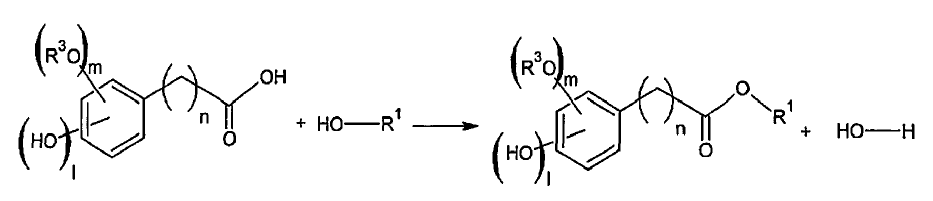 , and in at least one second step b) the at least one phenolic acid ester and at least one sterol R²-OH undergo an enzymatic transesterification using a lipase to at least one steryl phenolate according to reaction scheme (IV)

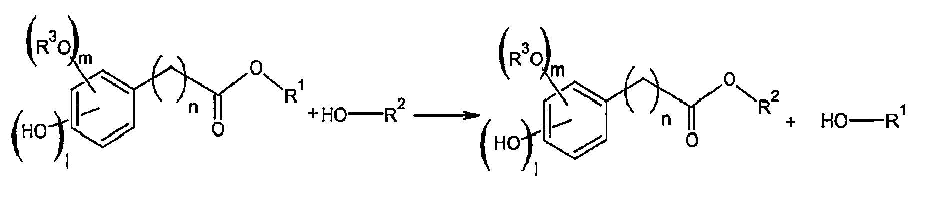

## Description

The present invention relates to a method for obtaining a phenolic ester according to claim 1, a method for obtaining a steryl phenolate according to claims 6 and 15.

### Description

Ferulic acid belongs to the class of phenolic acids, which are secondary plant metabolites and powerful, hydrophilic antioxidants present particularly in vegetables, fruits, spices and grains. In addition to their antioxidant activity, phenolic acids show anti-inflammatory, anti-carcinogenic, UV protecting, antimicrobial and neural protective effects.

A drawback of the widespread use of phenolic acid is their limited solubility in oil based systems, in which they could fulfill their antioxidant activity. The esterification of the acid group with an alcohol, such as a free sterol, leads to a molecule having an increased solubility in fats and oils but still keeping their high antioxidant capacity. An increased lipophilicity provides the possibility to use these esterified phenolic acids as antioxidants in foods and cosmetics. So far only the esters of gallic acid and parabene are authorized as food additives. Ferulate esters are so far only used for UV filter in skin conditioner or as antioxidants.

Phenolic acids can be lipophilized through a covalent attachment of a fatty alcohol to the acid group. For instance, ferulic acid esters can be obtained chemo-enzymatically under harsh conditions with methanol using para-toluene sulfonic acids as catalysts. These ferulic acid methyl esters are then transesterified with the desired fatty alcohol enzymatically.

Different approaches for direct enzymatic esterification of phenolic acids, for instance by using lipases, have been described in the past. Lee et al. (Biotechnology Letters, 2006, 28; 581-585) describes the esterification of para-methoxy cinnamic acid with 2-ethyl hexanol and ferulic acid with ethanol using an immobilized lipase from *Candida antarctica* (Novozyme® 435). The esterification of ferulic acid with ethanol reached almost full conversion in 48 hours in isooctane. Similar results were obtained by Vosmann et al. (Solvent-free lipase catalyzed preparation of long-chain alkyl phenylpropanoates and phenylpropyl alkanoates, Journal of Agricultural and Food Chemistry, 2006, 54: pages 2969-2976). Here, a solvent-free system was used at 80 mbars to esterify ferulic acid with 9-octadecen-1-ol reaching the yield of 98% in 48 hours. Other lipases such as a lipase from *Mucor miehei* (Lipozyme®) were much less investigated in the past on their ability to esterify ferulic acid directly. Using the lipase from *Mucor miehei* no efficient esterification (i.e. high yield in short time) of ferulic acid with alcohols and in particular with fatty alcohols has been demonstrated before. Generally, the esterification in non-polar solvents is most efficient. But the solubility of the ferulic acid is limited in such systems. Thus, there is still a need for an efficient enzymatic method for enzymatic esterification of a phenolic acid with an alcohol.

Steryl ferulates are esters of various plant sterols connected through an ester linkage to a ferulic acid. In nature, steryl ferulates appear mainly in the bran of grains such as rice, wheat or corn. Steryl ferulates are known to have different beneficial health effects such as cholesterol lowering properties and antioxidant activities. Therefore, they have a high potential as food additives. Steryl ferulates from rice bran called γ-Oryzanol are available on the market; however, the sterol profile of this mixture may not be ideal for the antioxidant activity. In any case γ-Oryzanol can be bought in large quantities and fairly reasonable purities. But due to the extraction from rice the sterol pattern is specific for rice and there is a clear lack of single steryl ferulates on the market.

Plant sterols appear naturally in free form or conjugated to a fatty acid, a sugar or phenolic acid. The esterification with fatty acids and sterols has been investigated before. Weber and coworkers (Fatty Acid Steryl, Stanyl and Steroid-Esters by Esterification and Transesterification in Vacuo using Candida Rugosa Lipase as Catalyst, Journal of Agricultural and Food Chemistry, 2001, 49: page 67 to 71) explored the esterification activity of sitosterol with fatty acids under reduced pressure in a solvent-free system. In another study the esterification of plant sterols with lauric acid using Novozyme 435 in n-hexane was shown to provide a yield of 79.3% (He et al., Lipase Catalyzed Synthesis of Phytostanyl Esters in Non-Aqueous Media, Journal of Molecular Catalysis B: Enzymatic, 2010, 67: page 60 to 65; WO 01/53511 A1).

The enzymatic esterification of plant sterols with phenolic acids has only been demonstrated rarely on the other hand. For instance, Weber et al. showed the transesterification of ethyl dihydrocinnamate with cholesterol using Lipozyme. However, dihydrocinnamic acid has a saturated side chain and no hydroxyl group on the aromatic ring and possesses therefore no antioxidant activity. Although its structure is close to phenolic acids it does not belong to the group of phenolic acids, which possess at least one hydroxyl group on the aromatic ring. The enzymatic synthesis of steryl ferulates has been described using *Candida rugosa* lipase and vinyl ferulate as substrate (Tan and Shahidi, Chemoenzymatic Synthesis of Phytosteryl Ferulates and Evaluation of Their Antioxidant Activity, Journal of Agricultural and Food Chemistry, 2011, 59: pages 12375-12383; Chigorimbo-Murefu et al., J. Mol. Cat. B: Enzymatic, 2009, 277-282). However, the vinyl ferulate used as phenolic acid ester substrate had to be chemically synthesized using mercury acetate what would require a purification effort in order to apply the obtained sitosteryl ferulate in a food system. The lipase from *Candida rugosa* was shown to be the only lipase able to catalyze the transesterification.

So far the transesterification of a phenolic acid alcohol ester with the exception of vinyl ferulate has not been described. Thus, there is still a need for providing enzymatic methods for obtaining steryl phenolates (phenolic acid esterified with a sterol).

This object is being solved by the methods described in the following.

Accordingly, a first method is provided, which enables the synthesis of at least one phenolic acid ester by enzymatic esterification of at least one phenolic acid with at least one alcohol R¹-OH in a reaction according to reaction scheme (I) 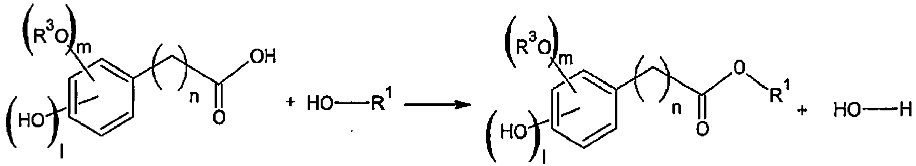 wherein
- R¹ is a saturated C₁-C₃₀ alkyl or non-saturated C₃-C₃₀ alkyl,
- n = 0-10, preferably 2-5; wherein in case n ≥2 at least one double bond may be included in the carboxylated alkyl side chain,
- R³ is a saturated C₁-C₅ alkyl or non-saturated C₂-C₅ alkyl,
- m = 0-4, preferably 1-3,
- l = 1-3, preferably 1,
wherein the esterification is catalyzed by at least one lipase and/or at least one esterase,
wherein the reaction temperature is between 25°C and 80°C, preferably between 50°C and 70°C, most preferably between 60°C and 65°C,
wherein the reaction time is between 20 and 100 h, preferably between 50 and 80 h, most preferably between 52 and 72 h,
wherein the phenolic acid concentration is between 1 and 10 mg/ml, preferably between 1.5 and 8 mg/ml, most preferably between 2 and 5 mg/ml,
wherein the reaction is carried out in an organic solvent,
wherein the alcohol R¹-OH is used in a ratio to the solvent between 1 µl/ml and 100 µl/ml, and
wherein the enzyme to phenolic acid ratio is 500 to 10 U, preferably 190 to 63 U of enzyme /g of phenolic acid.

1 U corresponds thereby to the amount of enzyme which sets free 1 µmol stearic acid per minute at pH 8.0 and 70°C (tristearin as substrate).

The present first method allows for a direct enzymatic esterification of phenolic acids with alcohols, in particular primary alcohols in good yields. The main factors affecting the esterification yield are substrate concentration, temperature, reaction time and enzyme-substrate ratio. When applying the conditions as outlined above molecular conversions between 55 and 95 %, preferably between 65 and 90 %, most preferably between 70 and 85 % were achieved. The conversion can vary depending on the primary alcohol used. For instance long chain alcohols such as decanol showed a somewhat better conversion than short chain alcohols such as ethanol.

In contrast to the method described by Stamatis et al. (J. Mol. Cat.B:Enzymatic, 2001, 11, 323-328), who describes the esterification of various cinnamic acids with octanol as reactant and solvent, the present method uses an organic solvent thus allowing the esterification with long chain alcohols.

The advantage of the present method is that it allows the use of of multiple alchols whereas according to Lee et al. (Biotechnology Letters, 2006, 28; 581-585) only ethanol is used. Stamatis et al. (J. Mol. Cat.B:Enzymatic, 2001, 11, 323-328) uses a solvent free system in octanol, whereas the present system can accommodate all common alcohols as listed. The alcohol used has an extremely high impact on the solubility of the products, and therefore the more diverse, the better the applicability. Also, the length of the alcohol will affect the localization of the alkyl phenolate in multiphase systems (emulsions etc.), which has a major impact on the antioxidant activity (if they are in the oil phase, in the water phase or on the interface of the two phases). Thus, the present method allows the use of a plurality of different also long chain alcohols allowing for the first time a large scale industrial application of the esterification of phenolates.

In an embodiment of said first method the moiety R¹ of the alcohol R¹-OH is a saturated C₂-C₂₅, preferably C₂-C₂₀ alkyl, most preferably C₂-C₁₀ alkyl or non-saturated C₄-C₂₅, preferably C₄-C₂₀ alkyl, most preferably C₄-C₁₀ alkyl. The moiety R¹ can be for instance a linear or branched C2-, C3-, C4-, C6-, C8-, C10-, C12-, C14-, C16- or C18-alkyl, wherein ethyl, n-propyl, n-butyl, n-hexyl-, n-octyl-, n-decyl, n-tetradecyl and n-octadecyl are mostly preferred. It is also possible to use a mixture of different alcohols, such as a mixture of ethanol, n-propanol, n-butanol, n-hexanol and decanol.

The number of OH-groups on the aromatic ring is between 1 and 3, wherein 1 OH-group is preferred. Said OH-groups of the phenolic acid may be present at any of the possible position of the aromatic ring, i.e. in ortho-, meta- or para-position to the carboxylated alkyl side chain, preferably in ortho- and/or para-position.

In a further embodiment of the above first method the moiety R³ as part of the phenolic acid is a saturated or non-saturated C₁-C₄ alkyl, preferably methyl or ethyl. The moiety R³ may include at least one double bond, may be functionalized with a hydroxy group, ether group, amino group, thiol group or similar groups. Thus, the aromatic moiety of the phenolic acid may be substituted with an alkoxy group (OR³), which can be further functionalized. A methoxy or alkoxy group is however mostly preferred.

The number of alkoxy groups OR³ on the aromatic moiety can be between 1 and 3, preferably 1 or 2. The alkoxy group OR³ can be in ortho-, meta- or para-position in respect to the carboxylated alkyl side chain, preferably in ortho- and/or- meta-position in respect to the carboxylated alkyl side chain.

In a further embodiment the carboxylated alkyl side chain comprises 2 to 5 carbon atoms (i.e. n = 2-5. It is preferred if n = 2. In case of n ≥ 2 (i.e. at least 2 carbon atoms) said side chain may include at least one double bond. The carboxylated side chain may be a propionate side chain or an acrylate side chain.

In more preferred embodiments the phenolic acid used as substrate comprises one or two OH-groups and one or two methoxy groups, wherein at least one OH-group is arranged in para-position to the carboxylated alkyl side chain.

The phenolic acids used as present may be selected from a group comprising salicylic acid, p-hydroxybenzoic acid, protocatechuic acid, gentisic acid, vanillic acid, veratric acid, gallic acid, syringic acid, o-, m-, p- coumaric acid, p-coumaroyl quinic acid, caffeic acid, dihydrocaffeic acid, ferulic acid, dihydroferulic acid, sinapic acid and rosmarinic acid. The preferred phenolic acid is ferulic acid.

In a further embodiment of above first method the at least one lipase or esterase is of eukaryotic origin, preferably a lipase from a yeast or a fungi. The lipase may be used in an immobilized form. In such case the lipase may originate from *Candida antarctica, Mucor miehei* or *Candida rugosa.* It is also possible to use a lipoprotein lipase or a cholesterol esterase. The most preferred lipase is an immobilized lipase from *Mucor miehei* (also known as Lipozyme®).

It also in general conceivable to use lipases of prokaryotic origin such as from *Pseudomonas fluorescens* or *Burkholderia cepacia.*

It is furthermore conceivable to apply esterases available from *Aspergillus niger, Fusarium oxysporum, Sporofrichum thermosporum, Talaromyces stipitatus, Clostridium thermocellum.* Typical esterases would be Ferulic acid esterases (EC 3.1.1.73).

As mentioned above the present esterification method of at least one phenolic acid is carried out in an organic solvent. Said organic solvent may be selected from a group comprising hexane, cyclohexane, octane, toluene, ethylacetate, DMSO, iso-octane, heptane, diethylether, chloroform, acetone, butanone, acetonitrile, dichloromethane or THF.

The preferred organic solvent is n-hexane, which may be dehydrated before use for instance by using a molecular sieve.

As pointed out above, the reaction conditions are specifically chosen in order to obtain a high conversion and yield. In further embodiments the reaction temperature may be between 60°C and 65°C, most preferably at 61°C; the reaction time may be between 52 and 72 h; and/or the phenolic acid concentration may be between 1.5 and 8 mg/ml, most preferably between 2 and 5 mg/ml.

It is furthermore preferred if the alcohol R¹-OH is used in a ratio to the organic solvent between 1 µl/ml and 75 µl/ml, and if the enzyme to phenolic acid ratio is 500 U - 10 U, preferably 190 U - 63U of enzyme/g of phenolic acid. The enzyme to phenolic acid ratio may also be described as being 5 to 2 (wt/wt), most preferably 3 to 1 (wt/wt). A typical enzyme:phenolic acid ratio 2.5 g/g, depending on the enzyme activity.

The phenolic acid concentration used can depend on the chain length of the alkyl chain of the alcohol used, for example when ethanol is used the preferred phenolic acid concentration is about 1.25 mg/ml, whereas when decanol is used the preferred phenolic acid concentration is about 2.8-3 mg/ml.

When using ferulic acid as phenolic acid and ethanol and decanol as alcohol R¹-OH, the preferred reaction conditions in said first method are as follows:
- reaction temperature of 61 °C, reaction time of 72 h, ferulic acid concentration 1.25 mg/ml, ethanol concentration 17 µl/ml, enzyme:substrate (ferulic acid) ratio of 2.5 g/g, dehydrated n-hexane as organic solvent (molecular conversion 79-80%);
- reaction temperature of 61°C, reaction time of 72 h, ferulic acid concentration 2.8 mg/ml, decanol concentration 75 µl/ml, enzyme:substrate (ferulic acid) ratio of 2.5 g/g, dehydrated n-hexane as organic solvent (molecular conversion 88%).

The present first method provides thus an efficient approach to obtain phenolic acid esters, such as ferulic acid esters, with good conversion rates. This allows for an esterification of phenolic acid in an industrial scale opening up the large-scale production of such esters.

As previously mentioned phenolic acid esters may be used as starting material for the enzymatic synthesis of steryl phenolates (phenolic acid esterified with a sterol). Thus, a method for obtaining at least one steryl phenolate is provided which comprises an enzymatic transesterification reaction of at least one phenolic acid ester and at least one sterol R²-OH in a reaction according to reaction scheme (II) 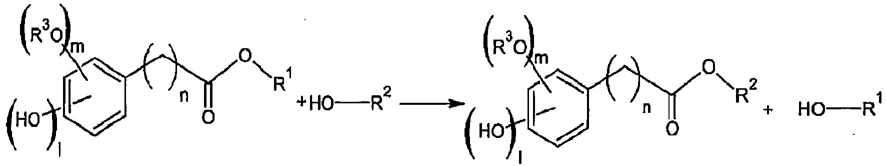 wherein
- R¹ is a saturated C₁-C₃₀ alkyl or non-saturated C₃-C₃₀ alkyl,
- R² is a sterol moiety,
- n = 0-10; preferably 2-5, wherein in case n ≥2 at least one double bond may be included in the esterified alkyl side chain,
- R³ is a saturated C₁-C₅ alkyl or non-saturated C₂-C₅ alkyl,
- m = 0-4, preferably 1-3,
- l = 1-3, preferably 1,
wherein the transesterification is catalyzed by at least one lipase and/or at least one esterase.

The present second method allows for a direct enzymatic transesterification of phenolic acid esters with sterols in good yields. Factors affecting the transesterification yield are substrate concentration, temperature, reaction time and enzyme-substrate ratio. When applying the reaction conditions as described below an overall yield for the transesterification of a phenolic acid ester with at least one sterol may reach 40 - 80 %, preferably 50-70%, most preferably 55 to 65 % (amount of sterol converted to steryl phenolate).

A transesterification of vinyl ferulate with a sterol such as sitosterol, dihydrocholesterol or 5α-androstane-3β,17β-diol using *Candida rugosa* lipase is specifically not covered by the present method. Thus, vinyl ferulate is exempted as a substrate. This has the advantage that a cumbersome clean-up of the vinyl ferulate as phenyl ester (which is required since vinyl ferulate is chemically synthesized using mercury acetate) is avoided. A further advantage of exempting vinyl ferulate is that the product can be used in the food sector. When using vinyl ferulate the vinyl group is released after transesterification as formaldehyde. Formaldehyde is however known to be toxic and harmful. This hampers the use of vinyl ferulate tremendously.

In an embodiment of said first method the moiety R¹ is a saturated C₂-C₂₅, preferably C₂-C₂₀ alkyl, most preferably C₂-C₁₀ alkyl or non-saturated C₄-C₂₅, preferably C₄-C₂₀ alkyl, most preferably C₄-C₁₀ alkyl. The moiety R¹ can be for instance a linear or branched C2-, C3-, C4-, C6-, C8-, C10-, C12-, C14-, C16- or C18-alkyl, wherein ethyl, n-propyl, n-butyl, n-hexyl-, n-octyl-, n-decyl, n-tetradecyl and n-octadecyl are mostly preferred.

As previously pointed out the number of OH-groups on the aromatic ring is between 1 and 3, wherein one OH-group is preferred. Said OH-groups of the phenolic acid may be present at any of the possible position of the aromatic ring, i.e. in ortho-, meta- or para-position to the carboxylated alkyl side chain, preferably in ortho- and/or para-position.

In a further embodiment of the above second method the moiety R³ (as in the first method) as part of the phenolic acid is a saturated or non-saturated C₁-C₄ alkyl, preferably methyl or ethyl. The moiety R³ may include at least one double bond, may be functionalized with a hydroxy group, ether group, amino group, thiol group or similar groups. Thus, the aromatic moiety of the phenolic acid may be substituted with an alkoxy group (OR³), which can be further functionalized. A methoxy or alkoxy group is however mostly preferred.

As described for the first method likewise in the second method the number of alkoxy groups OR³ on the aromatic moiety can be between 1 and 3, preferably 1 or 2. The alkoxy group OR³ can be in ortho-, meta- or para-position in respect to the carboxylated alkyl side chain, preferably in ortho- and/or- meta-position in respect to the carboxylated alkyl side chain.

In a further embodiment the esterified alkyl side chain comprises 2 to 5 carbon atoms (i.e. n = 2-5. It is preferred if n = 2. In case of n ≥ 2 (i.e. at least 2 carbon atoms) said side chain may include at least one double bond. The esterified side chain may be a propionate side chain or an acrylate side chain.

In more preferred embodiments the phenolic moiety or two OH-groups and one or two methoxy groups, wherein at least one OH-group is arranged in para-position to the esterified alkyl side chain.

The phenolic acids esters used in the second transesterification method may be selected from a group comprising esters of salicylic acid, p-hydroxybenzoic acid, protocatechuic acid, gentisic acid, vanillic acid, veratric acid, gallic acid, syringic acid, o-, m-, p- coumaric acid, p-coumaroyl quinic acid, caffeic acid, dihydrocaffeic acid, ferulic acid, dihydroferulic acid, sinapic acid and rosmarinic acid. The preferred phenolic acid ester is a ferulic acid ester, in particular a ferulic acid ethyl ester.

In a preferred embodiment the sterol R²-OH used in the second method is selected from a group comprising sitosterol, campesterol, stigmasterol, brassicasterol, stigmastadienol, dihydrositosterol, sitostanol, campestanol, delta-7-avenasterol, cycloartenol, 24-methylenecycloartanol and delta-5-avenasterol. It is also possible that a mixture of sterols R²-OH is used, for example a mixture of sitosterol, sitostanol and campesterol. Such a mixture may comprise 75.6% sitosterol, 12.2% sitostanol, 8.1 % campesterol and 4.1 % other minor phytosterols.

As in the first method different lipases and/or esterases may be applicable also in the second method for enzymatic transesterification. The at least one lipase or esterase may be of eukaryotic origin, preferably a lipase from a yeast or a fungi. The lipase may be used in an immobilized form. In such case the lipase may originate from *Candida antarctica, Mucor miehei* or *Candida rugosa.* It is also possible to use a lipoprotein lipase or a cholesterol esterase. The most preferred lipase is lipase from *Candida rugosa.*

It also in general conceivable to use lipases of prokaryotic origin such as from *Pseudomonas fluorescens* or *Burkholderia cepacia.*

It is furthermore conceivable to apply esterases available from *Aspergillus niger, Fusarium oxysporum, Sporotrichum thermosporum, Talaromyces stipitatus, Clostridium thermocellum.* Typical esterases would be Ferulic acid esterases (EC 3.1.1.73)

In an embodiment of the second method the sterol R²-OH concentration used is between 1 and 10 mg/ml, preferably between 2 and 8 mg/ml, most preferably between 3 and 5 mg/ml. For example 3.7 mg/ ml (11 mg/ 3 ml) sterol may be used.

In another variant of the second method the enzyme to sterol ratio is between 200,000 U and 6,000 U enzyme /g sterol, preferably between 150,000 U and 6,000 U enzyme/g sterol, most preferably between 100,000 U and 18,000 U enzyme/g sterol. It is also possible to define the enzyme/sterol ratio as 10 : 1 (wt/wt), preferably 5 : 2 (wt/wt), most preferably 3 : 1 (wt/wt).

1 U corresponds thereby to the amount of enzyme which sets free 1 µmol fatty acid per minute at pH 7.2 and 37°C (trigylceride as substrate).

It is furthermore preferred in the second method if the molar ratio of phenolic acid ester to sterol (molar/molar) is 5 : 1, preferably 3 : 1, most preferably 2.5 : 1.

The reaction time of the second method may be between 24 and 200 h, preferably between 48 and 192 h, most preferably between 48 and 120 h.

The reaction temperature of the second method may be between 25°C and 80°C, preferably 50°C and 70°C, most preferably between 60°C and 65°C, even most preferably at 63°C.

The present second transesterification method of at least one phenolic acid ester with at least one sterol is preferably carried out in an organic solvent. Said organic solvent may be selected from a group comprising hexane, cyclohexane, octane, toluene, ethylacetate, DMSO, iso-octane, heptane, diethylether, chloroform, acetone, butanone, acetonitrile, dichloromethane or THF.

The most preferred conditions for the transesterification reaction may be 63°C, 3g/g enzyme:sterol ratio, 3.7 mg/ ml (11 mg / 3 ml) sterol concentration in hexane (water free hexane), phenolic acid ester: sterol ratio (molar/molar) 2.5 : 1.

The first method for enzymatic esterification of a phenolic acid with an alcohol and the second method for enzymatic transesterification of a phenolic acid ester with a sterol may also be combined in a two-step process.

Thus, also a third method for obtaining at least one steryl phenolate from at least one phenolic acid in a two-step enzymatic process is provided, wherein
- in at least one first step a) the at least one phenolic acid and at least one alcohol R¹-OH undergo an enzymatic esterification to at least one phenolic acid ester according to reaction scheme (III) 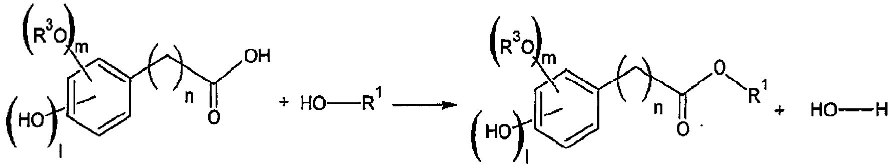 , and
- in at least one second step b) the at least one phenolic acid ester and at least one sterol R²-OH undergo an enzymatic trans-esterification to at least one steryl phenolate according to reaction scheme (IV) 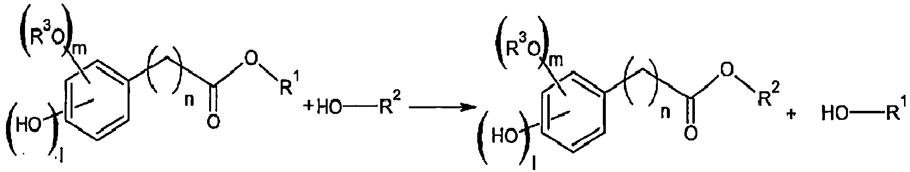 wherein
   R₁ is a saturated C₁-C₃₀ alkyl or non-saturated C₃-C₃₀ alkyl,
   R² is a sterol moiety,
   n = 0-10, preferably 2-5; wherein in case n ≥2 at least one double bond may be included in the respective carboxylated or esterified alkyl side chain,
   R³ is a saturated C₁-C₅ alkyl or non-saturated C₂-C₅ alkyl,
   m = 0-4, preferably 1-3,
   - l = 1-3, preferably 1,
   wherein the esterification and transesterification are catalyzed by at least one lipase and/or esterase, respectively.

The meaning of the moieties is here the same as above described.

In an embodiment of the present third method at least one first step a) comprises a method with the features of the first esterification method (reaction scheme I) as described in detail above. The obtained phenolic acid ester may undergo at least a partial purification. For example the enzyme is removed for instance by simple filtration and organic solvent and the alcohol used for esterification are evaporated to obtain a crude reaction product comprising as main product the corresponding phenolic acid ester. This dried residue may then be used without any further work-up for the subsequent transesterification step b).

In a further embodiment of the present third method the at least second step b) comprises a method with the features of the second transesterification method (reaction scheme II) as described in detail above.

The two-step synthesis of steryl phenolates allows a simple enzymatic synthesis of these valuable compounds, which can be used as food additives.

The present invention is explained in the following in more detail by means of several examples with reference to the figures. It shows:
- Figure 1: a calibration diagram for quantifying ferulate esters as phenolic acid esters, and
- Figure 2A,B: a diagram with a time course of molecular yield of ferulate esters with an alcohol mix.

### Example 1: Esterification of ferulic acid

### Chemicals and enzymes

Ferulic acid, ≥99% was provided by Sigma-Aldrich, Switzerland. Methyl ferulate, 99% and ethyl ferulate, 98% were purchased from Alfa Aesar, Switzerland. Wako Pure Chemical Industries, Japan delivered the Oryzanol, min. 98%. All used solvents were of HPLC grade. Lipozyme^{®}, immobilized lipase from *Mucor miehei,* >30 U/g (lot result: 63 U/g) was provided by Sigma-Aldrich, Switzerland. 1 U refers to the amount of enzyme which liberates 1 µmol stearic acid per minute at pH 8.0 and 70 °C.

### Enzymatic synthesis

For a typical ethyl ferulate synthesis experiment 3 mg of ferulic acid and 7.5 mg Lipozyme^{®} were weighed into a 10 ml glass tube with a Teflon-lined screw cap. 2.95 ml n-hexane and 50 µL ethanol were added and the solution was shaked thoroughly. The n-hexane used for the reaction was dehydrated over 4 A molecular sieve before use. The samples were incubated in an oven at 60°C for up to 72 h without shaking. Aliquot samples of 50 µl were collected during incubation, and the samples were evaporated under a gentle nitrogen stream at 50°C. After this the ferulates were redissolved in 500 µl solvent B (see HPLC-conditions below) and filtered through a 0.45 µm Teflon filter into a HPLC vial.

The synthesis of ethyl ferulate and decyl ferulate was optimized using surface response methodology. Different solvents were tested for the synthesis of ethyl ferulate. Based on the findings on optimal conditions for ethyl ferulate and decyl ferulate, optimal conditions for the other alcohols were derived based on the alcohol chain length. The number of carbon atoms was linearly associated with the ferulic acid and alcohol concentration.

### HPLC analysis and quantification

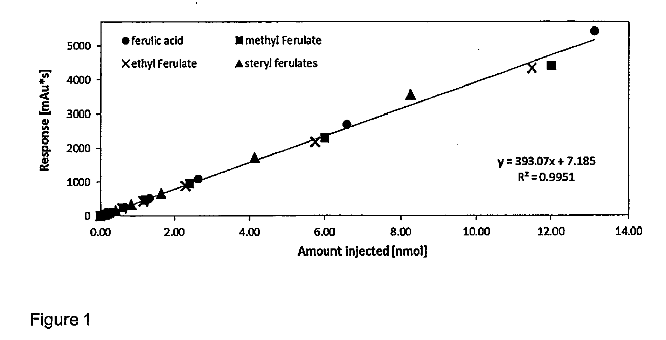

Ferulic acid and ferulate esters were analyzed using high performance liquid chromatography (HPLC, Agilent 1100, Switzerland). A reverse phase xBridge™Phenyl column from Waters with a particle size of 3.5 µm with gradient elution at 25°C was used for the separation of the ferulates. Solvent A was 1 % acetic acid in water and solvent B composed of acetonitrile, water, butanol, and acetic acid at a ratio of 88:6:4:2. The flow was set to 0.6 ml/min and a constant injection volume of 5 µl was applied. The elution program was in 3 min a linear gradient from 75:25 (A:B) to 100% B, isocratic flow of 100% B for 5 min, 4 min linear gradient to 75:25 (A:B) and 2 min isocratic 75:25 (A:B). For the analysis of dodecyl ferulate the isocratic flow of 100% B was extended to 11 min. Detection of the alkyl ferulates was achieved with a DAD detector at 325 nm. For the quantification of ferulic acid and ethyl ferulate external calibration was used (0.1-13 nmol/injection). For the quantification of the other esters ferulic acid, commercially available ferulate esters (methyl ferulate, ethyl ferulate, and steryl ferulate) were used to create a molar calibration curve (Figure 1). The UV response originates on the ferulic acid and is not significantly influenced by the alcohol esterified to it. Therefore, the quantification of all ferulate esters, except ethyl ferulate were calculated based on this calibration. Identification was supported by the specific UV spectra of ferulic acid. Additionally the reaction product identities were confirmed by mass spectrometry using a Synapt™ G2 time_of-flight mass spectrometer from Waters. The negative ionization mode was applied and the sample was introduced by direct infusion.

### Experimental design and surface response methodology

The design and evaluation of the optimization experiments was carried out using The Unscrambler X (CAMO Software, Oslo, Norway) using the Box-Behnken design model to fit the experimental data to a second-order polynomial equation. The variables used were time (24-72h), temperature (55-65°C), enzyme substrate ratio (1-4 g/g, i.e. 1-4 g of the immobilized enzyme preparation per gram of ferulic acid substrate), and contents of ferulic acid and alcohol. For the optimization of ethyl ferulate synthesis the ferulic acid content varied from 0.833 to 2.5 mg/ ml and the ethanol concentration varied from 8.33 to 25 µl/ ml. For the decyl ferulate synthesis the ferulic acid content was 1.67 to 5 mg/ ml and the decanol content from 25 to 75 µl/ ml. In both models (ethyl ferulate and decyl ferulate) all experiments were carried out once including a center sample, which was repeated six times. Optimal conditions were confirmed in triplicate analysis.

### Optimization of ethyl ferulate synthesis

The synthesis of phenolic acid esters is still graded as difficult, especially for 3-hydroxy cinnamic acids. Previous studies using Lipozyme^{®} for the esterification of ferulic acid showed only low yield in solvent free systems or other rather polar solvents. Although the solubility in hexane of ferulic acid is limited, the esterification seems to be possible due to a reasonable solubility of the product. Pretests (data not shown) showed low solubility of ethyl ferulate in hexane although with the addition of the alcohol which lead to the conclusion, that rather low concentrations have to be applied. Based on the pre-experiments also the parameters for the optimization model were chosen. For the optimization of the ethyl ferulate synthesis the second-order polynomial model could be fitted to the experimental data. The evaluation of the model showed a p-value of the lack of fit of 0.2, which indicates an adequate explanation of variance of the data. As well a high coefficient of determination of 0.95 indicates a good fitting of the experimental data to the model calculated. All linear and quadratic predictors have a significant influence. There are two regions where a yield above 70% can be reached. Both at 50 µl/3 ml ethanol for an enzyme substrate ratio equal and above 2.5 g/g and for rather low ferulic acid concentrations. There is no clear increase of yield when going from an enzyme substrate ratio of 2.5 to 4 g/g. But a clear trend towards a low ferulic acid concentration can be seen. The ethanol concentration seems to be crucial to not be too high or too low. Also a trend towards a maximum at medium incubation time can be observed. It is possible that after a certain time the reaction goes towards alcoholysis but for this system this does not seem convincing and needs to be confirmed. Optimal conditions of 61°C, 52h, 3.75 mg/3 ml ferulic acid, 57.5 µl/3 ml ethanol, and an enzyme substrate ratio of 2.5 g/g was investigated. In a second step the influence of the solvent was tested by applying the conditions mentioned above in hexane, cyclohexane, octane, toluene, butanone, acetone and solvent free in ethanol in duplicates. The yield detected after 52h were 67.1, 49.9, 51.3, 32.9 % for hexane, cyclohexane, octane and toluene respectively. In butanone, acetone and ethanol no ethyl ferulate could be detected. This corresponds well with former studies. The results show a clearly higher yield in hexane, for which solvent the synthesis was optimized. However, no higher yield than the one in hexane was observed. Due to the high influence of the ethanol concentration this factor was examined again by testing the following ethanol concentrations in triplicates: 42.5, 50, 57.2, 65 µl/3 ml. After 52h the reaction was stopped and the analysis of the synthesized ethyl ferulate showed molecular conversions of 65.1% ± 1.7, 72.1% ± 1.2, 67.1% ± 2.6, and 58.4% ± 2.7 were found. The predicted values for these conditions were 67.7, 72.4, 74.1, and 72.9%, respectively. Generally the values measured are somewhat lower and the optimum is slightly shifted. Therefore, the maximum conditions found are 61°C, 52h, 3.75 mg/3 ml ferulic acid, 50 µL/3 ml ethanol, and an enzyme substrate ratio of 2.5 g/g. The predicted value for these conditions is 72.3% and the actual value measured is 72.1% ± 1.2 which fits very well the model built based on the chosen experimental design. To check also the effect of a longer incubation up to 72h the optimal conditions chosen were applied again in triplicate. After 72h a molecular conversion of 79.2% ± 2.1 was detected. For these conditions the model predicts a yield of 71.9%. This experiment showed that the predicted decrease in yield for longer incubation times does not cover well the reality. In detail a longer incubation time does lead to a higher yield, also not drastically higher. The synthesis of ethyl ferulate using Lipozyme^{®} in hexane was optimized which makes a yield of 79% within 72h of incubation possible.

### Optimization of decyl ferulate synthesis

The optimization for ethyl ferulate synthesis was repeated due to essential differences in solubility and influence of alcohol concentration on the enzyme activity. Therefore, the same experimental design was applied as described above with adjusted ferulic acid and alcohol concentration. Again the model was fitted to the experimental data. This time the coefficient of determination was calculated to 0.91, which is somewhat lower than the one for the ethyl ferulate synthesis but still a good correlation of the calculated values to the measured data point can be observed. Also the p-value of the lack of fit is in this case lower, namely 0.08, which is just above significance level. This indicates an adequate explanation of variance by the model. For the optimization of decyl ferulate synthesis all linear factors except the ferulic acid concentration have significant impact on the yield. Concerning the enzyme substrate ratio a slight increase from 1 to 2.5 g/g can be recognized, mainly on the time scale. For the enzyme substrate ratio of 4 g/g only lower yields were measured. A higher enzyme substrate ratio does not seem necessary anyway since good yields can be reached already at smaller amounts. Unlike for the ethanol no clear optimum decanol concentration can be observed in the contour plots. It seems that the higher the decanol concentration the higher the yield. Based on the model optimal conditions of 61°C and 72h seem to be a good solution. The other factors are difficult to evaluate and need to be double checked. Therefore, some data points were repeated in triplicates. First the amount of enzyme was double checked using 8.5 mg/3 ml ferulic acid and 225 µl/3 ml decanol. The same enzyme substrate ratios were used as in the model. The found molecular conversions were 72.7% ± 11.9, 88% ± 2.6 and 78.7% ± 4.2 for the enzyme substrate ratios of 1, 2.5 and 4 g/g. This covers the findings for the ethyl ferulate synthesis that 2.5 g/g seems to be an optimal amount and less or more are counterproductive. After also the decanol concentration was varied finding no significant difference between 195, 225 255 µl/3 ml. A concentration of 225 µl/3 ml seems therefore acceptable. Finally also the ferulic acid concentration was evaluated using the concentrations 7, 8.5, 10 mg/3 ml which lead to a yield of 91% ± 2.2, 88% ± 2.6, and 82.2 % ± 3.1, respectively. Only a significant lower yield for the highest concentration could be found. Therefore optimal conditions of 61 °C, 72h, 8.5 mg/3 ml ferulic acid, 50 µl/3 ml ethanol, and an enzyme substrate ratio of 2.5 g/g were defined. The calculated yield for these conditions is 96.6% and the measured yield is 88% ± 2.6. The measured yield is significantly lower than the yield calculated based on the model. Generally can be said, that the synthesis of decyl ferulate is less sensitive to changing factors compared to the synthesis of ethyl ferulate but an optimum could still be found.

### Esterification of various alcohols

After the optimization of the ethyl ferulate and decyl ferulate synthesis other alcohols were tested as well. The concentrations applied were adjusted linearly from the optimal conditions found for C2 to C10 as described above. The concentrations for the esterification reaction with tetradecanol and octadecanol were equal to the ones for decyl ferulate. The temperature was kept constant at 61°C, reaction time was 72h and the enzyme substrate ratio was also kept at 2.5. Reactions were carried out in triplicate. The esterification yield increases from ethyl ferulate (79%±2.1) to hexyl ferulate (92%±5.2). The yield of the longer alcohols does not significantly differ and varies from 84-90%. Lipozyme^{®} was also tested on its ability to esterify ferulic acid with secondary alcohols as isopropanol and 2-octanol. The yields are drastically lower for the secondary alcohols. In detail 29%±1.1 and 11%±1.2 yield for isopropanol and 2-octanol, respectively were found. Generally can be said that using this process parameters all linear alcohols (from C2 on) can be directly esterified to ferulic acid.

### Esterification with alcohol mix

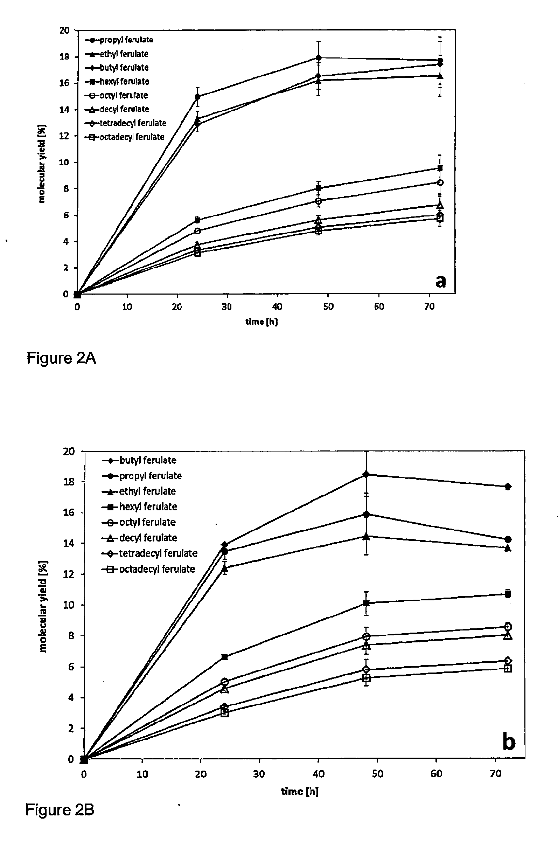

The esterification yield with longer alcohols was showed to be higher than for short alcohols. In an experiment with an alcohol mix, Lipozyme^{®} was tested on its alcohol preferences. Therefore, two experiments in triplicates were set up. In the first one the optimal conditions for ethyl ferulate synthesis were chosen in a second one the optimal conditions for decyl ferulate synthesis were installed. This reflects a higher ferulic acid concentration but a smaller molecular alcohol to acid ratio. The molecular concentration of all alcohols was the same and summed up equal to the optimal alcohol concentration. Only the linear alcohols were tested in competitions. The results from these experiments can be found in figures 2A,B. For both conditions the shorter alcohols, namely propanol, ethanol and butanol were preferably esterified. For the lower concentration the difference is even higher. At the higher ferulic acid concentration less ethyl and propyl ferulate but more decyl and hexyl ferulate is synthesized. In addition to that at the higher concentration the short ferulate esters show a tendency to alcoholysis after 48h.

In summary, the direct enzymatically esterification of ferulic acid was demonstrated. For ethyl ferulate and decyl ferulate the synthesis in hexane usinge Lipozyme^{®} was optimized which lead to maximal molecular conversions of 79.2%. ± 2.1 and 88% ± 2.6 after 72h were reached for ethyl ferulate and decyl ferulate, respectively. The optimal conditions found for ethyl ferulate are 61 °C, 72h, 3.75 mg/3 ml ferulic acid, 50 µl/3 ml ethanol, and an enzyme substrate ratio of 2.5 g/g. For the synthesis of decyl ferulate 61 °C, 72h, 8.5 mg/3 ml ferulic acid, 50 µl/3 ml ethanol, and an enzyme substrate ratio of 2.5 g/g were found to be optimal conditions. The main difference is therefore in the concentration of ferulic acid and alcohol. Based on these findings the esterification of ferulic acid with other alcohols such as linear propanol, butanol, hexanol, octanol, tetradecanol and octadecanol and the branched alcohols isopropanol and 2-octanol were tested. All linear alcohols were esterified in an expected extent, which is increasing from ethyl ferulate to hexyl ferulate and then stays constant until the 18 carbons long ester of ferulic acid.

### Example 2: Two step enzymatic synthesis of steryl ferulates from ferulic acid

### Chemicals and enzymes

Sigma-Aldrich, Switzerland provided the Ferulic acid, ≥99% and the β-sitosterol, ≥70% (impurities being mainly campesterol and β-sitostanol). Oryzanol, min. 98% was purchased from Wako Pure Chemical Industries, Japan. Alfa Aesar, Switzerland delivered the ethyl ferulate, 98%. All used solvents were of HPLC grade. Lipozyme^{®}, immobilized lipase from *Mucor miehei,* >30 U/g (1 U sets free 1 µmol stearic acid at pH 8.0 and 70 °C per minute) was from Sigma-Aldrich, Switzerland. The lipase of *Candida rugosa* type VII, ≥11.6 U/mg (at pH 7.2 and 37 °C one unit will hydrolyze 1.0 microequivalent of fatty acid from a triglyceride per minute) was also purchased at Sigma-Aldrich, Switzerland.

### Esterification of ferulic acid

Enzymatic synthesis of ethyl ferulate is described above. After incubation the samples were cooled to room temperature and filtered to remove the Lipozyme^{®}. Later the solvent was evaporated under nitrogen at 50°C. To ensure total ethanol evaporation a second time dry hexane was added and evaporated again. From the residue the transesterification could be performed.

### Transesterification

During the optimization commercial ethyl ferulate was used. For a typical transesterification reaction 5.4 mg ethyl ferulate and 5 mg sitosterol were placed in a 10 ml glass tube with a teflonated screw cap. Additionally 10 mg *Candida rugosa* lipase was weighed into the tube. After the addition of 1.5 ml of hexane, which was dried before over 4 A molecular sieve, the tube was shaken. The samples were incubated in an oven, standing.. For sample analysis after 48h, 120h and 192h, the tubes were put into cold water to cool down. After reaching room temperature, 50 µl of supernatant were transferred into another glass tube. The hexane was evaporated under a nitrogen stream at 50°C. Finally, the residue was re-dissolved in 500 µl solvent B (see HPLC conditions below) and filtrated.

### HPLC analysis and quantification

Steryl ferulates, ethyl ferulate and ferulic acid were analyzed using high performance liquid chromatography (Agilent 1100, Switzerland). For the separation a reverse phase xBridge Phenyl column from Waters with a particle size of 3.5 µm at 25°C was used. The detection was achieved at 325 nm with a DAD detector. Two solvents were used in a gradient, solvent A being 1% acetic acid in water and solvent B composed of 88:6:4:2 acetonitrile:water:butanol:acetic acid. A constant injection volume of 5 µl was applied and the flow was set to 0.6 ml/min. The elution sequence was composed of a 3 min linear gradient from 75:25 (A:B) to 100% B, isocratic flow of 100% B for 11 min, 4 min linear gradient to 75:25 (A:B) and 2 min isocratic flow 75:25 (A:B). For the quantification external standards of ethyl ferulate and oryzanol were used. Identification was reinforced by the specific UV spectra of ferulic acid. Molecular yield was calculated based on the amount steryl ferulates detected in the supernatant, which reflects the amount of sterols which were esterified.

The synthesis of ethyl ferulate was optimized as described above in Example 1. The transesterification was optimized using surface response methodology.

### Experimental design and surface response methodology

The Unscramble X from CAMO Software, Oslo, Norway was used to design the experiments and to evaluate the data. The optimization model used was the Box-Behnken design. The used variables were: sterol concentration (5-15 mg/3 ml), molecular substrate ratio (1-3 mole/mole), temperature (45-65°C), enzyme:sterol ratio phenolic acid ester to sterol (1-3 g/g). The 24 experiments were carried out once including a center sample, which was repeated three times. Optimal conditions were confirmed in triplicate analysis. The parameters and the ranges thereof were chosen based on preliminary experiments (data not shown). After 48 h, after 120h and after 192h samples were analyzed on their steryl ferulates content.

### Optimization of transesterification

To the experimental data the second-order polynomial equation was fitted for each time point. The model after 48h is very similar to the one after 120h. The main differences are that instead of the sterol concentration the substrate ratio is significant for the yield after 48h. And the interaction of sterol concentration and the substrate ratio loses its significance with the longer incubation but the temperature square factor gains significance. Generally the values for the factors are similar, the only thing drastically increasing is the β₀ value, which makes the calculated yield after 120h overall higher. Compared to the other two fittings, the β-coefficients for the yield after 192h have changed a lot. Only five factors are left which influence the result. The β₀ value is only slightly increased compared to the one after 120h. The lack of fit indicates a good fitting of the shape of the model compared to the experimental data, when being not significant. For the first two time points the lack of fit was calculated to 0.83 and 0.92 for 48h and 120h, respectively. Whereas after 192h the lack of fit was just above significance level with 0.08. For all time points the variance of the data was explained adequately with the fitted equations. For all models a high coefficient of determination was found of 0.97, 0.98 and 0.92 for the fitted model after 48h, 120h, and 192h, respectively. This indicates for all time points a good fitting of the data to the calculated yield based on the equations built.

All predictors are included in the calculation, also the non-significant ones. For the contour plot after 48h of incubation a clear tendency towards high temperature, high enzyme sterol ratio and high sitosterol concentration. The substrate ratio does show an increase from 1 to 2 but not necessarily from 2 to 3. Generally a yield of 40-50% should be reached after 48h.

Overall the yields are higher after 120h than after 48h but the same trends can be observed. Concerning the molar substrate ratio and the sitosterol concentration a trend towards the middle can be observed. Thus, leading to a highest calculated yield of 58.4% at the following conditions: 63°C, 3 g/g enzyme sterol ratio, 11 mg/3 ml sterol and a substrate ratio of 2.5.

Optimal conditions for an incubation time of 192h lead to a calculated yield of 63.2% at high temperature and high enzyme sterol ratio. But at lower sitosterol concentration (8.5 mg/3 ml) and substrate ratio (2.1 mole/mole). Generally only slightly higher yield can be reached by incubation for 192h than after 120h. Thus, 120h of incubation time seems to be suitable, applying the conditions mentioned above. In previous studies for the transesterification of vinyl ferulate with sterols using *Candida rugosa* lipase lead to a yield of 56% and 90%, respectively. The yield of around 60% is therefore well in the range which could be expected based on the comparison of the transesterification ability of ethyl ferulate compared to vinyl ferulate. The transesterification of ethyl ferulate with sterol using *Candida rugosa* lipase was successfully demonstrated and optimized.

### Two step synthesis of steryl ferulates

The fully enzymatic synthesis of steryl ferulates was confirmed. Therefore, the optimal conditions for the synthesis of ethyl ferulate as developed in Example 1 (61°C, 72h, 3.75 mg/3 ml ferulic acid, 50 µl/3 ml ethanol, and an enzyme substrate ratio of 2.5 g/g, in this case 42.5 µl/3 ml ethanol concentration) were applied. Therefore to synthesize 15.4 mg ethyl ferulate, around 18.5 mg ferulic acid is needed. This ferulic acid esterification was performed in triplicates. After the incubation the synthesized ethyl ferulate was quantified. The conversions of ferulic acid found were 81.2% ± 2.7 before evaporation. After evaporation the requested amount of sitosterol, enzyme, and hexane were added to reach the optimal conditions mentioned above (11 mg/3 ml sitosterol and a substrate ratio of 2.5). This step had two purposes, firstly to check the whole process in one and to double check the calculated yield mentioned above. For the transesterification the samples were incubated for 120h at 63°C and finally the concentration of steryl ferulates was determined. The calculated yield was 58.4% and the experimental yield was 58% ± 4 which corresponds very well. Therefore the optimization of the transesterification reaction of ethyl ferulate and sitosterol was successfully developed and a fully enzymatic synthesis of steryl ferulates was investigated.

The two-step, fully enzymatic synthesis of steryl ferulates was presented. In a first step the ferulic acid is esterified with ethanol using an immobilized lipase from *Mucor miehei* (Lipozyme^{®}) reaching a molecular conversion of around 81% in 72h. After the solvent is evaporated, the sitosterol, the new enzyme, and the new solvent can be added and the transesterification reaction can take place. Optimal conditions found for the transesterification step are the followings: 63°C, 3 g/g enzyme sterol ratio, 11 mg/3 ml sterol concentration and a substrate ratio of 2.5 mole/mole, leading to a yield of steryl ferulates of 58%. This process allows simple enzymatic synthesis for laboratory scale and also bears a great potential for industrial application.

## Claims

1. Method for obtaining at least one phenolic acid ester by enzymatic esterification of at least one phenolic acid with at least one alcohol R¹-OH in a reaction according to reaction scheme (I) 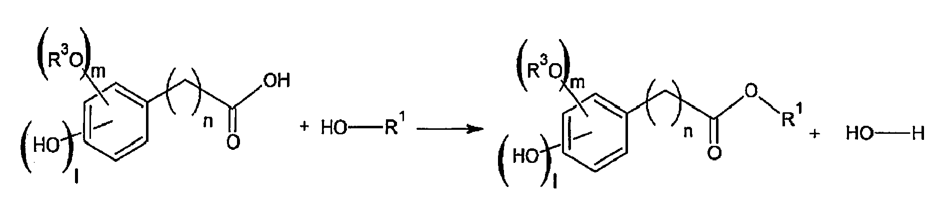 wherein
- R¹ is a saturated C₁-C₃₀ alkyl or non-saturated C₃-C₃₀ alkyl,
- n = 0-10, preferably 2-5; wherein in case n ≥2 at least one double bond may be included in the carboxylated alkyl side chain,
- R³ is a saturated C₁-C₅ alkyl or non-saturated C₂-C₅ alkyl,
- m = 0-4, preferably 1-3,
- I = 1-3, preferably 1
wherein the esterification is catalyzed by at least one lipase and/or at least one esterase,
wherein the reaction temperature is between 25°C and 80°C, preferably between 50°C and 70°C, most preferably between 60°C and 65°C,
wherein the reaction time is between 20 and 100 h, preferably between 50 and 80 h, most preferably between 52 and 72 h;
wherein the phenolic acid concentration is between 1 and 10 mg/ml, preferably between 1.5 and 8 mg/ml, most preferably between 2 and 5 mg/ml,
wherein the reaction is carried out in an organic solvent,
wherein the alcohol R¹-OH is used in a ratio to the solvent between 1 µl/ml and 100 µl/ml, preferably 1 µl/ml and 75 µl/ml, and
wherein the enzyme to phenolic acid ratio is 500 U - 10 U, preferably 190 U - 63 U of enzyme/g of phenolic acid.

2. Method according to claims 1, **characterized in that** R¹ is a saturated C₂-C₂₅, preferably C₂-C₂₀ alkyl, most preferably C₂-C₁₀ alkyl or non-saturated C₄-C₂₅, preferably C₄-C₂₀ alkyl, most preferably C₄-C₁₀alkyl.

3. Method according to claim 1 or 2, **characterized in that** R³ is a C₁-C₄ alkyl, preferably methyl or ethyl.

4. Method according to one of the preceding claims, **characterized in that** at least one lipase or esterase is of eukaryotic origin, preferably a lipase from a *Candida antarctica, Mucor miehei, Candida rugosa,* a lipoprotein lipase or a cholesterol esterase.

5. Method according to one of the preceding claims, **characterized in that** the organic solvent is selected from a group comprising hexane, cyclohexane, octane, toluene, ethylacetate, DMSO, iso-octane, heptane, diethylether, chloroform, acetone, butanone, acetonitrile, dichloromethane or THF.

6. Method for obtaining at least one steryl phenolate in an enzymatic transesterification reaction of at least one phenolic acid ester and at least one sterol R²-OH in a reaction according to reaction scheme (II) 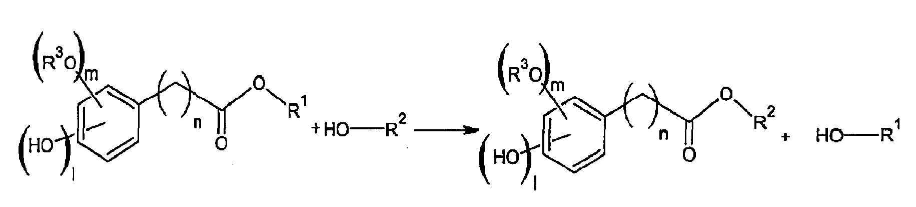 wherein
- R¹ is a saturated C₁-C₃₀ alkyl or non-saturated C₃-C₃₀ alkyl,
- R² is a sterol moiety,
- n = 1-10; preferably 2-5, wherein in case n ≥2 at least one double bond may be included in the esterified alkyl side chain,
- R³ is a saturated C₁-C₅ alkyl or non-saturated C₂-C₅ alkyl,
- m = 0-4, preferably 1-3,
- I = 1-3, preferably 1
wherein the transesterification is catalyzed by at least one lipase and/or at least one esterase.

7. Method according to claim 6, **characterized in that** the sterol R²-OH is selected from a group comprising sitosterol, campesterol, stigmasterol, brassicasterol, stigmastadienol, dihydrositosterol, sitostanol, campestanol, delta7-avenasterol, cycloartenol, 24-methylenecycloartanol and delta-5-avenasterol.

8. Method according to claim 6 or 7, **characterized in that** the sterol R²-OH is a mixture of sitosterol, sitostanol and campesterol.

9. Method according to one of the claims 6 to 8, **characterized in that** the sterol R²-OH concentration is between 1 and 10 mg/ml, preferably between 2 and 8 mg/ml, most preferably between 3 and 5 mg/ml.

10. Method according to one of the claims 6 to 9, **characterized in that** the enzyme to sterol ratio is between 200,000 U and 6,000 U enzyme /g sterol, preferably between 150,000 U and 6,000 U enzyme/g sterol, most preferably between 100,000 U and 18,000 U enzyme/g sterol

11. Method according to one of the claims 6 to 10, **characterized in that** the molar ratio of phenolic acid ester to sterol (molar/molar) is 5 : 1, preferably 3 : 1, most preferably 2.5: 1.

12. Method according to one of the claims 6 to 11, **characterized in that** the reaction time is between 24 and 200 h, preferably between 48 and 192 h, most preferably between 48 and 120 h.

13. Method according to one of the claims 6 to 12, **characterized in that** the reaction temperature is between 25°C and 80°C, preferably 50°C and 70°C, most preferably between 60°C and 65°C, even most preferably at 63°C.

14. Method according to one of the claims 6 to 13, **characterized in that** the transesterification reaction is carried out in an organic solvent, in particular in an organic solvent selected from a group comprising hexane, cyclohexane, octane, toluene, ethylacetate, DMSO, iso-octane, heptane, diethylether, chloroform, acetone, butanone, acetonitrile, dichloromethane or THF.

15. Method for obtaining at least one steryl phenolate from at least one phenolic acid in a two-step enzymatic process
**characterized in that**
- in at least one first step a) the at least one phenolic acid and at least one alcohol R¹-OH undergo an enzymatic esterification to at least one phenolic acid ester according to reaction scheme (III) 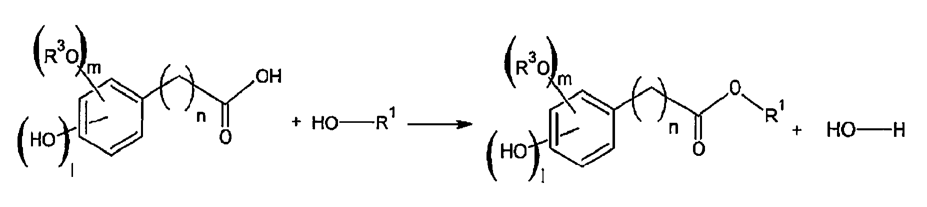 , and
- in at least one second step b) the at least one phenolic acid ester and at least one sterol R²-OH undergo an enzymatic trans-esterification to at least one steryl phenolate according to reaction scheme (IV) 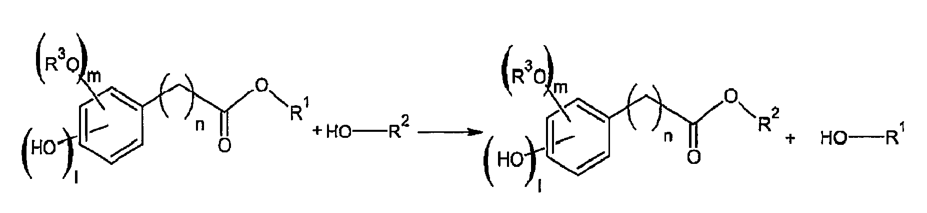 wherein
R₁ is a saturated C₁-C₃₀ alkyl,or non-saturated C₃-C₃₀ alkyl,
R² is a sterol moiety,
n = 1-10, preferably 2-5; wherein in case n ≥2 at least one double bond may be included in the respective carboxylated and esterified alkyl side chain,
R³ is a saturated C₁-C₅ alkyl or non-saturated C₂-C₆ alkyl,
m = 0-4, preferably 1-3.
- l = 1-3, preferably 1,
wherein the esterification and transesterification are catalyzed by at least one lipase and/or esterase, respectively.

16. Method according to claim 15, **characterized in that** the at least one first step a) comprises a method according to one of the claims 1 to 5.

17. Method according to claim 15 or 16, **characterized in that** the at least one second step b) comprises a method according to one of the claims 6 to 14.
